# EUROPEAN PATENT SPECIFICATION

(11) **EP 3 872 490 B1**
(45) Date of publication and mention of the grant of the patent: **08.03.2023**
(21) Application number: 19874812.1
(22) Date of filing: 10.10.2019
(51) Int. Cl.: G01N 33/53, G01N 21/64, G01N 21/78, G01N 33/536, B01L 3/00, G01N 33/543, G01N 33/58

(54) **CUP FOR IMMUNOASSAY, METHOD FOR PRODUCING SAME, AND IMMUNOASSAY METHOD**
BECHER FÜR IMMUNOASSAYS, VERFAHREN ZU DEREN HERSTELLUNG UND IMMUNOASSAY-VERFAHREN
GOBELET POUR IMMUNOESSAI, SON PROCÉDÉ DE PRODUCTION ET PROCÉDÉ D'IMMUNOESSAI

(30) Priority: 24.10.2018 JP 2018199985
(43) Date of publication of application: 01.09.2021
(73) Proprietor: Toppan Printing Co., Ltd., Tokyo 110-0016 (JP)
(72) Inventor: SHIOYA Toshihito, Tokyo 110-0016 (JP); SATO Hiromu, Tokyo 110-0016 (JP)
(74) Representative: TBK
(86) International application number: PCT/JP2019/040028
(87) International publication number: WO 2020/085104

(56) References cited:
- WO-A1-2011/012859
- WO-A1-2012/013959
- WO-A1-2012/121310
- WO-A1-2012/121310
- JP-A- 2012 157 267
- JP-A- 2015 023 822
- US-A1- 2002 187 515
- US-A1- 2002 187 515
- US-A1- 2014 323 330
- US-A1- 2017 165 662

## Description

### [Technical Field]

The present invention relates to an immunoassay cup for use in analysis of an immune system using an antigen-antibody reaction according to the preamble of claim 1 for examining various biomolecules, cancer cells, and the like, a corresponding method of producing the immunoassay cup, and a corresponding immunoassay method.

### [Background Art]

Antigens are substances that cause an immune response in a living body, and include pathogenic viruses, bacteria, pollen, eggs, wheat, and the like. Antibodies are collectively called immunoglobulins, which are proteins produced to eliminate antigens that enter the body. For example, vaccines include inactivated pathogenic bacteria or viruses, which are administered to promote production of antibodies against pathogens in the body and to acquire immunity against infectious diseases.

In the field of biological measurements such as detection of pathogenic bacteria or cancer cells, genetic analysis of DNA, and measurement of environmentally harmful substances, the type and quantity of antigens or the like are measured by measuring the binding between a biological substance to be measured (for example, an antigen) and an examination substance (for example, antibody) selectively binding to the biological substance to be measured by using an immunological reaction.

As described in NPL 1 below, a known method for examining the immune system typically involves directly or indirectly labeling the antibodies that capture antigens included in the specimen to be measured with a fluorescent dye, isotope, or other labeling substance (hereinafter, referred to as a marker), and measuring the amount of fluorescence or radiation from the marker after capture (after antigen-antibody reaction) for use in diagnosis.

As a marker used for measurement of antigens or the like, a fluorescent marker, which is more convenient to use than a radioisotope that requires strict control, is typically used. In optical methods using a fluorescent marker or the like, an antibody to which a fluorescent marker is added is bound to an antigen, and luminescence from the marker is measured to thereby measure and analyze the antigen. A typical method is an enzyme immunoassay (for example, enzyme-linked immunosorbent assay, ELISA) that uses fluorescence, which is generated by reaction of a fluorescent marker added to the antibody bound to the antigen with an enzyme in a reagent.

In order to facilitate observation of biomolecules and identify the biomolecules to be measured, gold colloid, microparticles, or the like together with the fluorescent marker are added to the respective biomolecules. Examples of the microparticles include polystyrene beads. By adding the fluorescent marker or microparticles to the biomolecules, molecules of a size difficult to observe with the resolution of an optical microscope can be visualized. Hereinafter, the fluorescent marker and the microparticles are collectively referred to as fluorescent beads, or simply as beads.

In the analysis using the antigen-antibody reaction for immunoassay as described above, a test tube type container is used for storing liquid such as blood (hereinafter, collectively referred to as blood) containing a specimen to be measured, fluorescent beads as a fluorescent marker, a reagent for promoting luminescence (color development) reaction, and the like (for example, see PTLs 1 and 2).

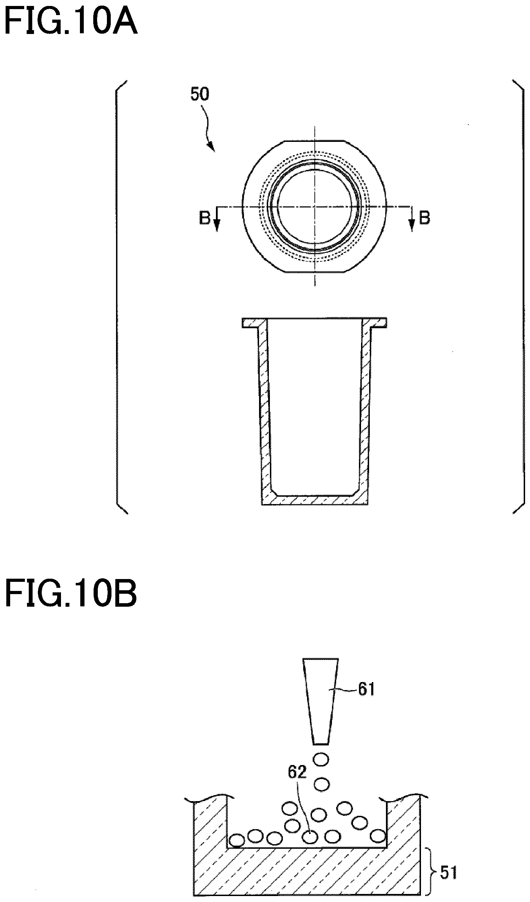

Fig. 10A illustrates a top view and a front cross-sectional view of a conventional immunoassay cup 50. Fig. 10B is a schematic cross-sectional view illustrating a state in which a plurality of fluorescent beads 62 are being added dropwise into the conventional immunoassay cup 50.

The conventional immunoassay cup 50 can be used for cell-level detection such as detection of cancer cells, or detection of large protein molecules (for example, molecular weight of 60 kDa or more). On the other hand, as seen from Fig. 10B, when a plurality of fluorescent beads 62 are added dropwise from a dropper tip 61 into the conventional immunoassay cup 50, the fluorescent beads 62 are irregularly arrayed. As a consequence, since the fluorescent beads 62 are dispersed in the liquid in which blood and the reagent are mixed, fluorescence is scattered and attenuated. Therefore, it is difficult to accurately measure and analyze proteins having a small molecular weight, such as cytokines having a molecular weight of approximately 5 to 60 kDa.

A generic fluorescent immunoassay cup according to the preamble of claim 1 is shown by PTL 3. Further conventional fluorescent immunoassay cups are disclosed by PTLs 4 to 6, which also show conventional methods of producing the fluorescent immunoassay cup. A conventional fluorescent immunoassay method using the fluorescent immunoassay cup is shown by PTL 7.

### [Citation List]

### [Patent Literature]

PTL 1: JP 4 095 176 B
PTL 2: JP 4 522 434 B
PTL 3: US 2002/187515 A1
PTL 4: WO 2012/013959 A1
PTL 5: WO 2011/012859 A1
PTL 6: US 2017/165662 A1
PTL 7: WO 2012/121310 A1

### [Non-Patent Literature]

NPL 1: Simple Immunology, pp. 195-201, published by Nankodo Co., Ltd. (2001)

### [Summary of the Invention]

### [Problem to be Solved by the Invention]

The present disclosure has been made to solve the above problem and aims to provide an immunoassay cup that enables accurate and reproducible measurement and analysis of proteins or the like having a small molecular weight such as cytokines, a method of producing the immunoassay cup, and an immunoassay method.

### [Solution to Problem]

The problem is solved by a fluorescent immunoassay cup according to claim 1, by a method of producing the fluorescent immunoassay cup according to claim 9, and by a fluorescent immunoassay method according to claim 10, respectively. Further advantageous developments of the present invention are set out in the dependent claims.

The present disclosure, which does not define the claimed subject-matter, includes the following.
[1] A fluorescent immunoassay cup for use in analysis of an immune system using an antigen-antibody reaction, in which beads are used as fluorescent markers, the fluorescent immunoassay cup including:
   a bottom; and
   a side wall connected to the bottom, wherein
   the bottom is provided with a plurality of pores each configured to accommodate a corresponding one of the beads, and
   a region between the pores on an upper side of the bottom is formed in a planar shape.
[2] The fluorescent immunoassay cup according to [1] is preferred, wherein the bottom and the side wall are integrally formed.
[3] The fluorescent immunoassay cup according to [1] or [2] is preferred, wherein an inner surface of the side wall includes a curved portion in a cross-section of the fluorescent immunoassay cup taken perpendicular to the bottom,
   an inner diameter of the side wall decreases toward the bottom, and
   the inner surface of the side wall is connected to the upper side of the bottom by a spline curve in the cross-section.
[4] The fluorescent immunoassay cup according to any one of [1] to [3] is preferred, wherein the inner surface of the side wall is hydrophobic.
[5] The fluorescent immunoassay cup according to any one of [1] to [4], wherein the upper side of the bottom and the side wall of the pores are connected by a spline curve in a cross-section of the fluorescent immunoassay cup taken perpendicular to the bottom.
[6] The fluorescent immunoassay cup according to any one of [1] to [5] is preferred, wherein the inner surface of the pores has been subjected to hydrophilic processing.
[7] The fluorescent immunoassay cup according to any one of [1] to [6] is preferred, further including a reinforcement rib protruding from the inner surface of the side wall.
[8] The fluorescent immunoassay cup according to any one of [1] to [7] is preferred, further including a first light-shielding portion that covers an outer surface of the side wall.
[9] The fluorescent immunoassay cup according to any one of [1] to [8] is preferred, further including a second light-shielding portion, wherein
   the second light-shielding portion is disposed on a surface of the bottom on a side opposite to that in which the pores are formed, and is located in a region that overlaps the side wall but does not overlap the pores.
[10] A method of producing the fluorescent immunoassay cup according to any one of [1] to [9], the method including the steps of:
   attaching a mold for forming the pores to a first mold;
   combining the first mold with a second mold to form a cavity defined between the first mold and the second mold; and
   injecting a resin into the cavity for molding.
[11] A fluorescent immunoassay method using the fluorescent immunoassay cup according to any one of [1] to [9], the method comprising the steps of:
   storing each of the beads into a corresponding one of the plurality of pores;
   introducing a liquid, which contains a specimen as a detection target substance, and a reagent, into the plurality of pores;
   pouring a sealing material into the fluorescent immunoassay cup to seal an upper part of the plurality of pores; and
   measuring fluorescence emitted from the pores.
   The present disclosure includes the following.
[12] A fluorescent immunoassay cup for use in analysis of an immune system using an antigen-antibody reaction, in which beads as fluorescent markers (fluorescent beads) are used, wherein
   an entirety of the fluorescent immunoassay cup including a bottom and a side wall are integrally formed,
   an upper side of the bottom has a planar shape in cross-sectional view, and
   a plurality of pores for accommodating each of the fluorescent beads are arrayed in a portion of the planar shape.
[13] The fluorescent immunoassay cup according to [12], wherein an inner surface of the side wall includes a curved portion in cross-sectional view, the side wall having an inner diameter decreasing toward the bottom, and
   the inner surface of the side wall is connected to the upper side of the bottom by a spline curve in cross-sectional view.
[14] The fluorescent immunoassay cup according to [12] or [13], wherein the inner surface of the side wall is hydrophobic.
[15] The fluorescent immunoassay cup according to any one of [12] to [14], wherein the upper side of the bottom and the side wall of the pores are connected by a spline curve.
[16] The fluorescent immunoassay cup according to any one of [12] to [15], wherein the inner surface of the pores has been subjected to hydrophilic processing.
[17] A method of producing the fluorescent immunoassay cup according to any one of [12] to [16], the method including the steps of:
   attaching a mold for forming the pores to a first mold;
   combining the first mold with a second mold to form a cavity defined therebetween; and injecting a resin into the cavity for molding.
[18] A fluorescent immunoassay method using the fluorescent immunoassay cup according to any one of [12] to [16], the method comprising the steps of:
   storing each of the fluorescent beads into a corresponding one of the plurality of pores;
   introducing a liquid, which contains a specimen as a detection target substance, and a reagent, into the plurality of pores;
   pouring a sealing material into the fluorescent immunoassay cup to seal an upper part of the plurality of pores; and
   measuring fluorescence emitted from the pores.

### [Advantageous Effects of Invention]

The present invention provides an immunoassay cup that enables accurate and reproducible measurement and analysis of small molecular weight proteins such as cytokines, a method of producing the immunoassay cup, and an immunoassay method.

### [Brief Description of Drawings]

Fig. 1A illustrates a top view of an immunoassay cup according to an embodiment of the present invention and a cross-sectional view of the immunoassay cup taken along the line A-A.
Fig. 1B is an enlarged microscopic image of a cross-section of a bottom of the immunoassay cup according to an embodiment of the present invention as viewed in an oblique direction.
Fig. 1C is a schematic cross-sectional view of pores in the immunoassay cup according to an embodiment of the present invention taken along the line A-A of Fig. 1A.
Fig. 1D is a schematic cross-sectional view of an immunoassay cup according to another embodiment of the present invention.
Fig. 2 is a schematic cross-sectional view illustrating a state in which a plurality of fluorescent beads are being added dropwise into an immunoassay cup according to an embodiment of the present invention.
Fig. 3 is a schematic cross-sectional view illustrating a specific shape of the immunoassay cup according to an embodiment of the present invention taken along the line A-A of Fig. 1A, and a state in which the beads are being accommodated in the pores provided in the bottom.
Fig. 4 is a schematic view illustrating an inner shape of the immunoassay cup (excluding the pores) in the schematic cross-sectional view of Fig. 3.
Fig. 5 is a schematic cross-sectional view illustrating a specific shape of the bottom of the immunoassay cup according to an embodiment of the present invention taken along the line A-A of Fig. 1A, a state in which beads, blood, and reagents are confined in the pores in the bottom and fluorescence is being measured.
Fig. 6A is a schematic cross-sectional view illustrating a step in a method of producing an immunoassay cup according to an embodiment of the present invention.
Fig. 6B is a schematic cross-sectional view illustrating a step in a method of producing an immunoassay cup according to an embodiment of the present invention.
Fig. 6C is a schematic cross-sectional view illustrating a step in a method of producing an immunoassay cup according to an embodiment of the present invention.
Fig. 6D is a schematic cross-sectional view illustrating a step in a method of producing an immunoassay cup according to an embodiment of the present invention.
Fig. 7A is a schematic cross-sectional view of an immunoassay cup according to still another embodiment of the present invention.
Fig. 7B is a schematic cross-sectional view of an immunoassay cup according to still another embodiment of the present invention.
Fig. 7C is a bottom view of an immunoassay cup according to still another embodiment of the present invention.
Fig. 8A is a microscopic image illustrating a light-shielding portion of an immunoassay cup according to still another embodiment of the present invention.
Fig. 8B is a microscopic image illustrating a light-shielding portion of an immunoassay cup according to still another embodiment of the present invention.
Fig. 9A is a top view of a sample stage according to still another embodiment of the present invention.
Fig. 9B is a schematic cross-sectional view of an adapter according to still another embodiment of the present invention.
Fig. 10A illustrates a top view of a conventional immunoassay cup and a cross-sectional view of the immunoassay cup taken along the line A-A.
Fig. 10B is a schematic cross-sectional view illustrating a state in which a plurality of fluorescent beads are being added dropwise into a conventional immunoassay cup.

### [Description of Embodiments]

With reference to the drawings, an immunoassay cup according to embodiments of the present invention, a method of producing the same, and an immunoassay method will be described below. Unless otherwise specified, for the sake of convenience, the same components are denoted by the same reference signs. Throughout the drawings, the thickness and the ratio of components may be exaggerated and the number of components may be reduced for convenience of illustration.

### [First Embodiment]

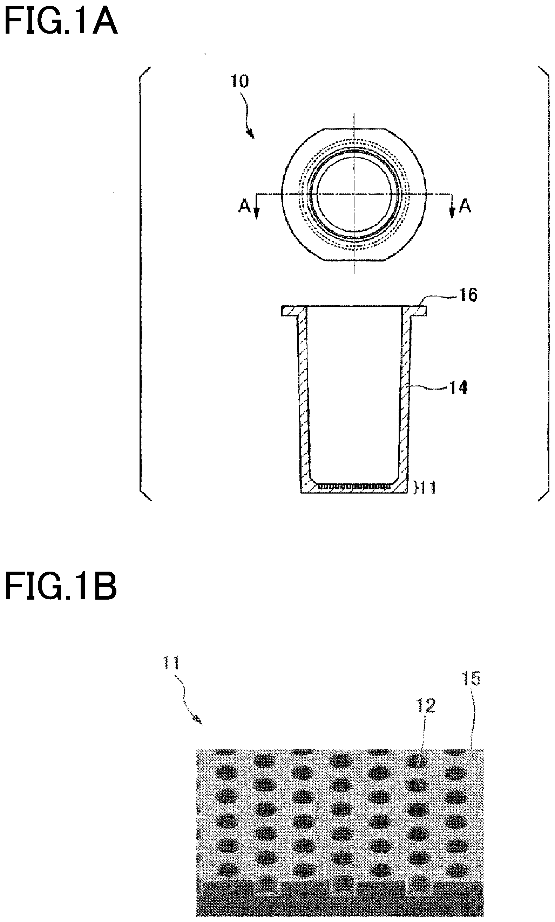

An immunoassay cup according to an embodiment of the present invention will be described. Fig. 1A illustrates a top view of an immunoassay cup according to an embodiment of the present invention and a cross-sectional view of the immunoassay cup taken along the line A-A. Fig. 1B is an enlarged microscopic image of a cross-section of a bottom of the immunoassay cup according to an embodiment of the present invention as viewed in an oblique direction. An immunoassay cup of the present embodiment is a fluorescent immunoassay cup for use in analysis of an immune system using an antigen-antibody reaction, in which beads are used as fluorescent markers, the fluorescent immunoassay cup including: a bottom; and a side wall connected to the bottom, wherein the bottom is provided with a plurality of pores each configured to accommodate a corresponding one of the fluorescent beads, and a region between the pores on an upper side of the bottom is formed in a planar shape.

An immunoassay cup 10 of the present embodiment includes a bottom 11 and a side wall 14. The side wall 14 may be integrally formed with the bottom 11. The bottom 11 has a plurality of pores 12 arrayed in the portion having the planar shape, and each pore 12 is configured to accommodate a corresponding fluorescent bead. A region between the pores 12 on an upper side 15 of the bottom is formed in a planar shape. The immunoassay cup 10 may further include a grip 16 on an upper end of the side wall 14. Hereinafter, the fluorescent beads may be simply referred to as beads.

The immunoassay cup 10 includes a substantially circular bottom 11 and a substantially tubular side wall 14. The shape of the bottom 11 is not specifically limited, and may also be a perfect circle, an ellipse, a rectangle, a square, a polygon, or the like. The shape of the side wall 14 is not specifically limited, and may be a tubular shape connected to the shape of the bottom 11. For example, the side wall 14 may have a circular or rectangular tubular shape.

The grip 16 protrudes from the upper end of the side wall 14 in a direction substantially parallel to the bottom 11. Due to the grip 16 provided on the immunoassay cup 10, the immunoassay cup 10 can be easily handled even when it is used with an automated handling apparatus such as a robot. Further, when the immunoassay cup 10 is housed in an adapter of a measurement system during immunoassay, the grip 16 serves as a stopper. Therefore, a distance between the pores 12 as the measurement site and a measurement apparatus in the measurement system can be optimized.

An area of the bottom 11 is not specifically limited, and may be, for example, in a range of 10 to 30 mm². A distance from the bottom 11 of the immunoassay cup 10 to the upper end of the side wall 14 (height of the immunoassay cup 10) is not specifically limited, and may be, for example, in a range of 10 to 15 mm.

The maximum thickness of the bottom 11 is preferably in a range of 0.2 mm to 0.6 mm, and more preferably 0.2 mm to 0.3 mm. When the maximum thickness of the bottom 11 is 0.6 mm or less, a decrease in fluorescence sensitivity due to autofluorescence of the material of the immunoassay cup 10 is less likely occur. When the maximum thickness of the bottom 11 is 0.2 mm or more, the mechanical strength of the immunoassay cup 10 is maintained.

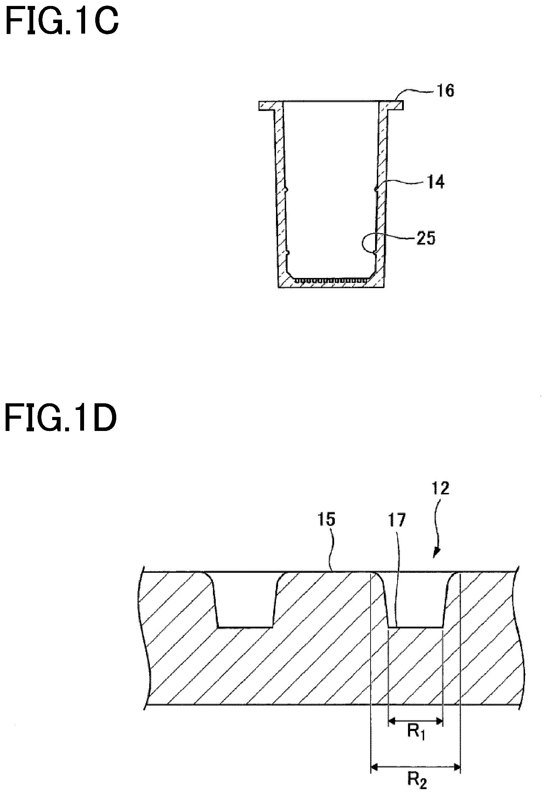

The thickness of the side wall 14 is preferably approximately the same as the thickness of the bottom 11, which is for example in a range of 0.2 mm to 0.6 mm, and more preferably 0.2 mm to 0.3 mm. Further, as shown in Fig. 1C, an inner surface of the side wall 14 may have a thickened portion, that is, a reinforcement rib 25. The reinforcement rib 25 is a portion protruding from the inner surface of the side wall 14. The reinforcement rib 25 may be formed in an annular shape extending along the inner periphery of the side wall 14. The number of reinforcement ribs 25 is not specifically limited, and, for example, one to four reinforcement ribs 25 may be provided, and preferably two or three reinforcement ribs 25 are provided. The thickness of the reinforcement ribs 25, that is, the dimension of the reinforcement rib 25 from the outer surface of the side wall to the inner surface of the side wall may be larger than the thickness of a portion of the side wall 14 other than the reinforcement rib 25, and may be, for example, in a range of 0.1 mm to 0.2 mm.

Due to the reinforcement rib 25 provided on the immunoassay cup 10, the mechanical strength can be increased even when the side wall 14 is thin as described above. For example, the immunoassay cup 10 does not easily collapse even when immunoassay is performed with an automatic handling apparatus such as a robot.

The planar shape of the pore 12 when the bottom 11 is viewed from above may be a circular or polygonal shape, and the minimum diameter of the pores is preferably 1.1 to 1.9 times, more preferably 1.2 to 1.8 times, and still more preferably 1.3 to 1.7 times the maximum diameter of the bead. When the minimum diameter of the pore 12 is 1.1 to 1.9 times the maximum diameter of the bead, each pore 12 does not accommodate two beads, but always accommodates one bead.

In immunoassay, the shape of the beads used with the immunoassay cup 10 of the present embodiment may also be a spherical shape. In general, the maximum diameter of the bead is in a range of 2 µm to 5 µm.

In view of the maximum diameter of the bead, the minimum diameter of the pore 12 may be in a range of 2.2 µm to 9.5 µm, preferably 2.4 µm to 9.0 µm, and more preferably 2.6 µm to 8.5 µm. For example, for 3 µm beads, the minimum diameter of the pore 12 is preferably in a range of 3.3 µm to 5.7 µm, more preferably 3.6 µm to 5.4 µm, and still more preferably 3.9 µm to 5.1 µm.

The pore 12 preferably has a tapered shape with a diameter decreasing from the opening toward the bottom. Fig. 1D is a schematic cross-sectional view of pores in the immunoassay cup according to an embodiment of the present invention taken along the line A-A of Fig. 1A. The diameter of a pore bottom 17 of the pore 12 is denoted by R₁. The diameter of the opening of the pore 12 is denoted by R₂. The opening diameter R₂ of the pore 12 is a minimum diameter of the opening periphery of the pore 12 in a plane which is the same as the upper side 15 between the pores 12. The pore 12 preferably has a tapered shape having R₁ < R₂. Such a tapered shape of the pores 12 facilitates accommodation of the beads into the pores 12.

When the pore 12 has a tapered shape, R₁ is preferably 1.1 to 1.5 times, and preferably 1.2 to 1.4 times the maximum diameter of the bead. R₂ is preferably 1.6 to 1.9 times, and preferably 1.7 to 1.8 times the maximum diameter of the bead. When R₁ is 1.1 to 1.5 times the maximum diameter of the bead, the beads that have entered the pores 12 are not likely to leave the pores 12. When R₂ is 1.6 to 1.9 times the maximum diameter of the bead, each bead is likely to be accommodated in a corresponding pore 12.

When the pore 12 has a tapered shape, R₁ is preferably in a range of 2.2 µm to 7.5 µm, and more preferably 2.4 µm to 7.0 µm. R₂ is preferably in a range of 3.2 µm to 9.5 µm, and more preferably 6.8 µm to 9.0 µm. When R₁ is in a range of 2.2 µm to 7.5 µm in terms of maximum diameter of the bead, the beads that have entered the pores 12 are not likely to leave the pores 12. When R₂ is in a range of 3.2 µm to 9.5 µm in terms of maximum diameter of the bead, each bead is likely to be accommodated in a corresponding pore 12.

For example, when the bead diameter is 3 µm, R₁ is preferably in a range of 3.3 µm to 4.5 µm, and more preferably 3.6 µm to 4.2 µm. R₂ is preferably in a range of 4.8 µm to 5.7 µm, and more preferably 5.1 µm to 5.4 µm.

The depth of the pore 12, that is, the dimension from the upper side 15 between the pores 12 to the pore bottom 17 is preferably 1.1 to 1.5 times, and more preferably 1.1 to 1.3 times the maximum diameter of the bead. When the depth of the pore 12 is 1.1 to 1.3 times the maximum diameter of the bead, each bead is likely to be accommodated in a corresponding pore 12.

The depth of the pore 12 is preferably in a range of 2.2 µm to 7.5 µm, and more preferably 2.2 µm to 6.5 µm. When the depth of the pore 12 is in a range of 2.2 µm to 7.5 µm, each bead is likely to be accommodated in a corresponding pore 12. For example, when the maximum diameter of the bead is 3 µm, the depth of the pore 12 is preferably in a range of 3.3 µm to 4.5 µm, and more preferably 3.3 µm to 3.9 µm.

The number of pores 12 provided in the bottom 11 is, for example, preferably in a range of 180,000 to 270,000, and more preferably 200,000 to 250,000. When the number of pores 12 is 180,000 or more, sufficient fluorescence can be obtained from the immunoassay cup 10, which contributes to improvement in measurement accuracy. When the number of pores 12 is 270,000 or less, the adjacent pores 12 are not located too close to each other, which contributes to improvement in performance of the immunoassay cup 10.

The pores 12 are regularly arranged on the bottom 11. The pores 12 are preferably arranged in a close-packed array, in which equilateral triangles are formed by connecting the centers of adjacent pores 12. In this arrangement, the center-to-center distance between the adjacent pores 12 is preferably in a range of 6 µm to 12 µm, and more preferably 7 µm to 11 µm. When the distance between the adjacent pores 12 is in a range of 3 µm to 7 µm, a number of beads sufficient for immunoassay can be accommodated in the pores 12 in the bottom 11 of the immunoassay cup 10, and a decrease in the manufacturing yield of the immunoassay cups 10 can be prevented.

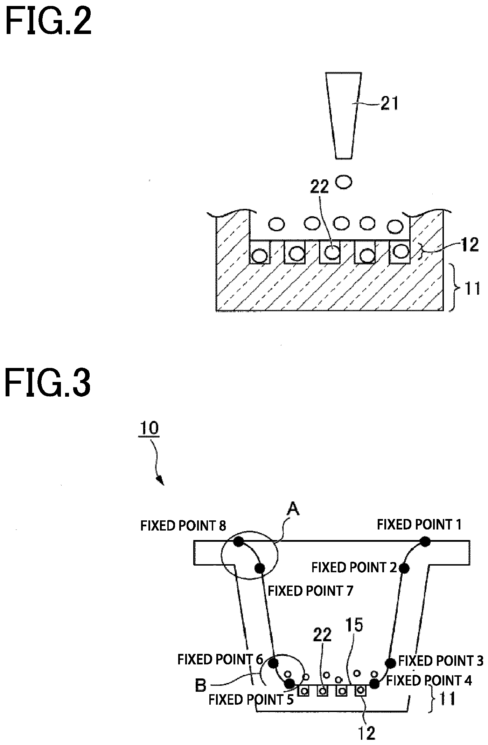

Fig. 2 is a schematic cross-sectional view illustrating a state in which a plurality of fluorescent beads are being added dropwise into an immunoassay cup according to an embodiment of the present invention. When beads 22 are added dropwise from a dropper tip 21 so that each bead is accommodated in a corresponding pore 12 to perform immunoassay as described later, the beads are prevented from being dispersed in a mixture of blood and reagents, unlike the case where the conventional immunoassay cup is used. Accordingly, the fluorescence emitted from the beads is not scattered and attenuated.

When the beads are each accommodated in respective pores 12 that are regularly arranged, such as in a linear manner, the fluorescence from the beads is measured by a measurement system via a condenser lens, for example. Therefore, using the immunoassay cup according to the present embodiment enables highly accurate and reproducible measurement and analysis of small molecular weight proteins such as cytokines.

Fig. 3 is a schematic cross-sectional view, illustrating a specific shape of the immunoassay cup 10 according to an embodiment of the present invention, taken along the line A-A of Fig. 1A, and shows a state in which the beads 22 are being accommodated in the pores 12 provided in the bottom 11. The bottom 11 and the side wall 14 of the immunoassay cup 10 are preferably integrally formed instead of being bonded to each other. Here, the description "the bottom and the side wall are integrally formed" means that the bottom and the side wall are seamlessly and continuously formed by injection molding or the like, which is distinguished from the case where the bottom and the side wall are independently formed and bonded to each other via an adhesive or the like. In the case where the bottom and the side wall are independently formed and bonded to each other via an adhesive or the like, high accuracy is required for adhesion of the bottom and the side wall. On the other hand, when the bottom 11 and the side wall 14 are integrally formed, an adhesion step is not required, so the yield of the immunoassay cup 10 is improved. Further, when the bottom and the side wall are independently formed and bonded to each other via an adhesive or the like, the adhesive is required to be made of a material that does not affect reagents, samples, and the like used in the immunoassay. On the other hand, when the bottom 11 and the side wall 14 are integrally formed, there is no need to consider an adhesive. It should be noted that the immunoassay cup 10 according to an embodiment of the present invention may be produced by adhering the bottom and the side wall that have been individually formed to each other as long as they do not depart from the gist of the present invention.

The material for the immunoassay cup 10 is preferably a polymer compound or a resin that can be subjected to injection molding in a manufacturing step as described later. When the immunoassay cup 10 is made of a hydrophobic material, the reagents, blood, and the like are not left on the side wall and are easily introduced into the pores. Further, since fluorescence is detected in immunoassay, a material having high light transmittance and low autofluorescence is preferred. In addition, since the immunoassay cup 10 is a disposable medical device, an inexpensive material is preferred. Examples of the above polymer compound and resin include aliphatic cyclic polyolefin (COP), polymethyl methacrylate resin (PMMA), and polystyrene (PS). The material for the immunoassay cup 10 is preferably COP (cyclic olefin polymer) due to its high light transmittance, low autofluorescence, hydrophobicity, and low cost.

The immunoassay cup 10 has a bowl shape (tapered shape) with the inner diameter thereof, that is, the inner diameter of the side wall 14, decreasing toward the bottom 11 (that is, a distance between a fixed point 2 and a fixed point 7 > a distance between a fixed point 3 and a fixed point 6).

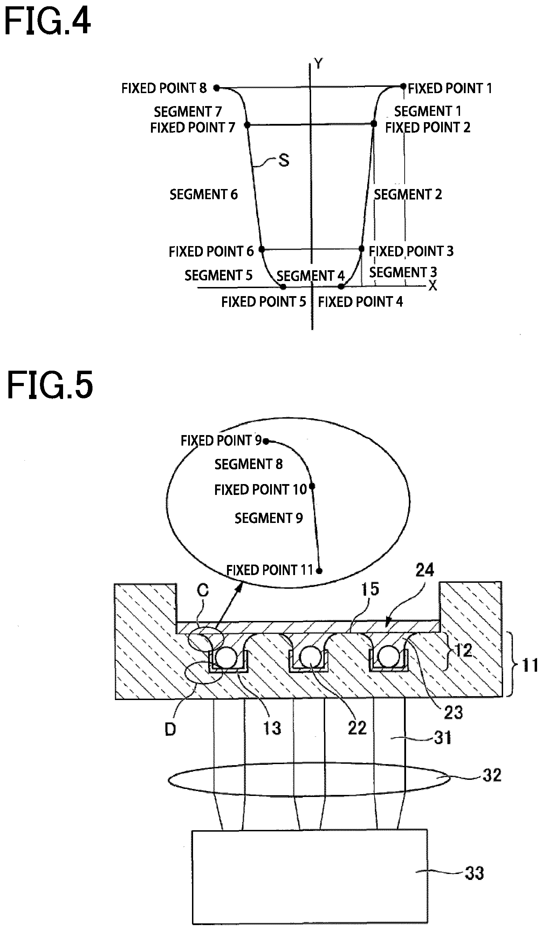

Fig. 4 is a schematic view illustrating an inner shape of the immunoassay cup 10 (excluding the pores) in the schematic cross-sectional view of Fig. 3. In Fig. 4, a region between the pores 12 on the upper side 15 of the bottom 11 is illustrated as being continuous for convenience of illustration. Preferably, in a cross-section of the fluorescent immunoassay cup 10 taken perpendicular to the bottom 11, an inner surface S (fixed points 1 to 4 and fixed points 5 to 8) of the side wall 14 of the immunoassay cup 10 includes a curved portion, and is connected to a straight portion on the upper side of the bottom (fixed points 4 to 5) by a spline curve in the above cross-section. More specifically, it is preferred that a spline curve, which includes a portion between the fixed points 8 and 7 and a portion between the fixed points 6 and 5 indicated by the ovals A and B in Fig. 3, respectively, (or a portion between the fixed points 1 and 2 and a portion between the fixed points 3 and 4) having a substantially arc shape and a portion between the fixed points 7 and 6 (or a portion between the fixed points 2 and 3) having a substantially straight shape is connected to a straight portion on the upper side of the bottom 11 (a portion between the fixed points 4 and 5).

The curve having a substantially arc shape indicated by the oval A facilitates introduction of the beads, blood, and reagents. Further, since the portion of the side wall 14 indicated by the oval A is curved and bent to form a flange (grip 16), the immunoassay cup 10 can be easily held. In addition, the curve having a substantially arc shape indicated by the oval B facilitates flow of the beads, blood, and reagents as described later. When the portion indicated by the oval B has a corner instead of an arc, the beads may be aggregated at the corner. As a consequence, the beads may fail to enter the pores 12, or the fluorescence intensity may increase at the corner, leading to variations in fluorescence intensity, which may reduce the sensitivity in detecting the measurement target. On the other hand, since the portion indicated by the oval B includes a curve having a substantially arc shape, the beads can be easily introduced into the pores 12 and reduce variations in fluorescence intensity.

In order to observe fluorescence on the underside of the immunoassay cup 10, a bottom having a smaller thickness is preferred. In other words, since the fluorescence emitted from the beads is observed via a surface of the bottom 11, on a side opposite to that in which the pores 12 are formed, the bottom 11 preferably has a minimum thickness. However, when the immunoassay cup 10 is produced by injection molding, it is preferred to uniformly cool the polymer compound or resin constituting the immunoassay cup 10 as a whole after injection molding of the immunoassay cup 10. Accordingly, the bottom 11 and the side wall 14 preferably have substantially the same thickness. Therefore, in order to maintain the strength of the side wall 14, a region of the bottom 11 where the pores 12 are formed is preferably limited to a straight portion in the bottom (that is, a portion of the bottom 11 having a flat upper surface). In other words, the pores 12 are preferably formed in a region of the bottom 11 other than the curved portion connected to the side wall 14.

Fig. 5 is a schematic cross-sectional view illustrating a specific shape of the bottom of the immunoassay cup according to an embodiment of the present invention, a state in which beads, blood, and reagents are confined in the pores in the bottom and fluorescence is being measured. In the schematic cross-sectional view shown in Fig. 5, the upper side 15 of the bottom 11 and the side wall of the pores 12 are preferably connected by a spline curve. Specifically, as indicated by the oval C in Fig. 5, the side wall of the pores 12 preferably forms a spline curve, which includes a portion between the fixed points 9 and 10 having a substantially arc shape, and a portion between the fixed points 10 and 11 having a substantially straight shape. With this configuration, the beads, which are typically spherical, are easily introduced into the pores 12 one by one. As described above, liquid such as blood containing a specimen to be measured may be hereinafter simply referred to as blood.

As shown in Figs. 4 and 5, preferably, the immunoassay cup 10 according to an embodiment of the present invention has a bowl shape (tapered shape), in which an opening for introducing beads, blood, and reagents (indicated by the oval A in Fig. 4), a transition part to the bottom 11 (indicated by the oval B in Fig. 4), and an inlet of the pore 12 (indicated by the oval C in Fig. 5, also referred to as an opening of the pore 12) have a substantially arc shape, and the remaining parts have a substantially straight shape, and they are connected to each other by a spline curve. Further, when the immunoassay cup 10 is made of a hydrophobic material, the side wall 14 has a hydrophobic inner surface, which causes liquid such as blood and reagents to smoothly flow into the pores 12 without being left on the side wall of the cup due to splashing or the like during injection of the liquid. Therefore, expensive reagents and blood can be effectively used without being wasted.

As shown in the oval D in Fig. 5, an angle formed between the side wall and the bottom of the pore 12 is preferably a right angle. In other words, in the cross-section taken perpendicular to the bottom of the pore 12, an angle formed between the side wall and the bottom of the pore 12 is preferably not rounded but has a straight corner. With this configuration, the beads that have entered the pores 12 with momentum can be prevented from leaving the pores 12.

Further, as indicated by reference number 13 in Fig. 5, at least the straight portion of the side wall of the pores 12 and the bottom of the pore 12, that is, the inner surface of the pore 12, is preferably processed to have hydrophilicity. The inner surface of the pore 12 is preferably coated with a hydrophilic material. With this configuration, liquid such as blood and reagents is easily accommodated in the pores 12. Preferably, a method of coating with a hydrophilic material includes covering a portion other than the pores 12 with a mask such as a stencil mask, and applying a hydrophilic spray or the like to the inner surface of the pores 12 over the mask.

In the following description, an immunoassay method using an immunoassay cup according to an embodiment of the present invention will be described. Fig. 5 illustrates a state in which the beads 22, blood, and a reagent 23 are confined in the pores 12 in the bottom of the immunoassay cup according to an embodiment of the present invention, and fluorescence 31 is measured by a measurement system 33 via a condenser lens 32.

The beads 22 are fine particles for capturing specimens to be measured. Specifically, examples of the beads 22 include gold colloid, polystyrene beads, and the like. The beads 22 have a surface that can adsorb an antibody (capture antibody) for capturing a measurement target substance (i.e., detection target substance) contained in the specimen to be measured.

Examples of the specimens to be measured include cells such as cancer cells, blood and the like. Examples of the measurement target substance contained in the specimen to be measured include proteins having a large molecular weight of approximately 60 kDa or more and proteins having a small molecular weight of approximately 5 to 60 kDa (that is, cytokines). According to the immunoassay cup 10 of an embodiment of the present invention, proteins having a small molecular weight of, in particular, approximately 5 to 60 kDa can be detected with high sensitivity. In the following description, an example specimen to be measured is hereinafter referred to as blood.

The reagent is one that is typically used in an immunoassay method, and may be, for example, in the case of ELISA, an antibody (primary antibody) specifically binding to the measurement target substance, an enzyme-labeled antibody (secondary antibody) specifically reacting with the primary antibody, an enzyme substrate, or the like. When the primary antibody is labeled with an enzyme, a secondary antibody may not be required. In the present embodiment, the enzyme substrate is preferably a fluorescent reagent.

In measurement, the beads 22 are first introduced into the immunoassay cup 10 to arrange the beads 22 in the pores 12. The capture antibody may be adsorbed to the beads 22 in advance, or may be adsorbed after the beads 22 are arranged in the pores 12. Then, blood and the reagent 23 are injected into the immunoassay cup 10.

After introduction of the blood and the reagent 23, excess beads 22, which do not enter the pores 12, are removed together with excess blood and reagent. The excess beads, blood, and reagent can be removed by pouring a sealing oil as a sealant into the immunoassay cup 10, and then tilting the immunoassay cup 10. That is, when the sealing oil is poured into the immunoassay cup 10, and the immunoassay cup 10 is then tilted, the beads 22 in the pores 12 coated with a hydrophilic material are fixed thereto and do not escape from the pores 12. However, other beads, excess blood, and excess reagent are removed by tilting the immunoassay cup 10.

As described above, the beads 22, blood, and the reagent 23 are confined and held in the pores 12 by a sealing material 24 (sealing oil) indicated by reference sign 24 in Fig. 5. Accordingly, the sealing material 24 individually seals a plurality of pores 12 to provide the pores 12 as independent reaction spaces. The material for the sealing material 24 is preferably one of fluorine-based oil and silicone-based oil, a mixture thereof, or the like.

According to the immunoassay cup 10 of an embodiment of the present invention, a plurality of independent reaction spaces defined by the pores 12 and the sealing material 24 can be formed. Therefore, fluorescence 31 generated by reaction between blood and the reagent is prevented from being interfered with by other beads 22 contained in the immunoassay cup 10.

Further, according to the immunoassay cup 10 in an embodiment of the present invention, the amount of blood and reagent 23 may be sufficient to fill all the spaces defined by the pores 12 and the sealing material 24. Since the pores 12 are minute spaces as described above, the amount of blood and reagent 23 can be reduced compared with the case where the conventional immunoassay cup is used. That is, according to the immunoassay cup 10 of an embodiment of the present invention, the intensity of fluorescence 31 generated by reaction between blood and reagent can be increased even when a small amount of the blood and the sample 23 are used.

The fluorescence 31 emitted from the pores 12 can be measured on the underside of the immunoassay cup (that is, a surface of the bottom 11 on a side opposite to that in which the pores 12 are formed) by using the measurement system 33. As the measurement system 33, for example, an inverted optical microscope and a fluorescence microscope can be used.

According to the immunoassay cup 10 of the present invention, the number of biomolecules to be observed can be specified based on the intensity of the fluorescence 31 generated by reaction between blood and reagent. In general, the resolution of an optical microscope is in the range of the wavelength of visible light, and is of the order of hundreds of nm. In the immunoassay method according to an embodiment of the present invention, biomolecules as the measurement target are labeled with a fluorescent dye, and the fluorescence can be detected with improved sensitivity by using the immunoassay cup 10 according to an embodiment of the present invention. Accordingly, a measurement target of a small molecular substance such as cytokine can be observed.

Alternatively, the fluorescence 31 can be measured and analyzed by using a communication device having a digital microscope or a digital camera and a calculating unit as the measurement system 33. A wavelength selection filter can be inserted in the optical path of the fluorescence 31 as necessary. Thus, information on the function and activity state of the biomolecule can be obtained.

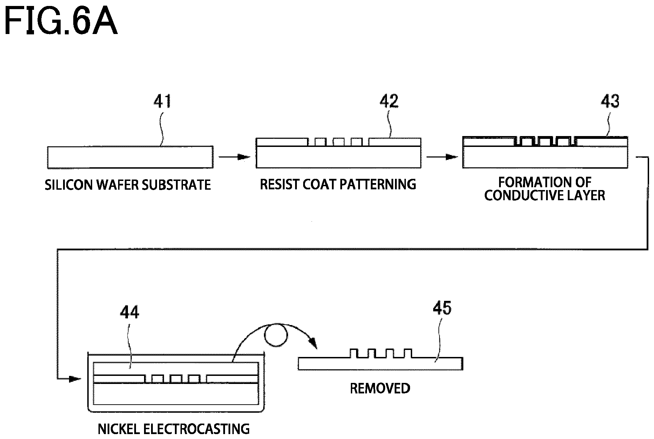

In the following description, a method of producing an immunoassay cup according to an embodiment of the present invention will be briefly described. Figs. 6A to 6D are schematic cross-sectional views each illustrating a step in a method of producing an immunoassay cup according to an embodiment of the present invention. First, as shown in Fig. 6A, a mold 45 for forming pores in the bottom of the immunoassay cup is fabricated. A method of fabricating a mold may be a known method for fabricating a thermal imprint mold. Fig. 6A illustrates an example of fabrication of a nickel mold by nickel electrocasting. First, a resist coat 42 is applied to a silicon wafer 41. The resist coat 42 is then exposed and developed to perform patterning of the resist coat 42. Then, a conductive layer 43 is formed on a surface of the resist coat and a portion of the silicon wafer 41 exposed by removing the resist coat 42. The nickel electrocasting is applied to the matrix thus formed to obtain a mold 45. The mold 45 has a plurality of projections corresponding to a plurality of pores of the immunoassay cup.

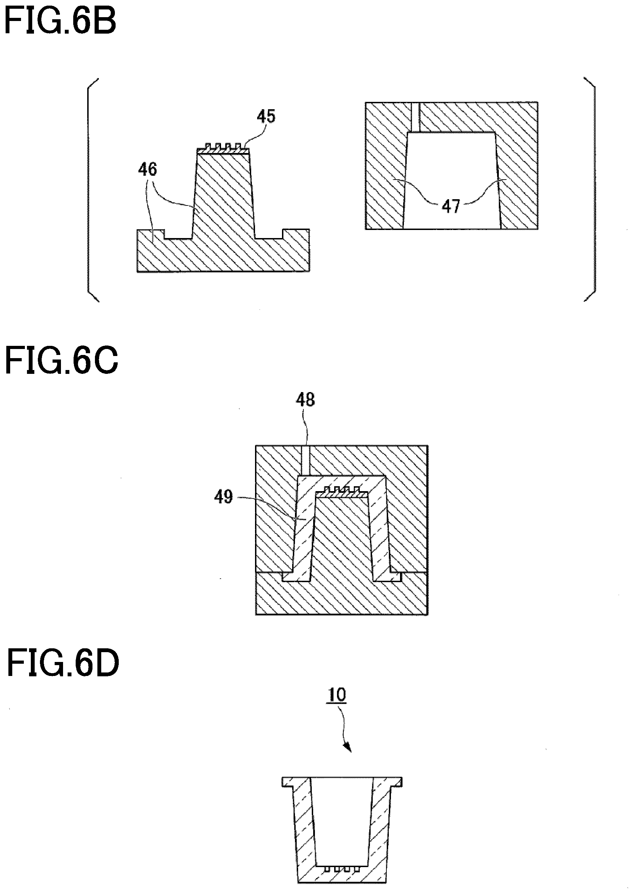

Then, as shown in Fig. 6B, the mold 45 is combined with a first mold 46. A method of combining the mold 45 and the first mold 46 is not specifically limited. The mold 45 and the first mold 46 can be fixed to each other in an injection molding apparatus, or can be attached via an adhesive or the like. Then, as shown in Fig. 6C, the first mold 46 is combined with a second mold 47. Accordingly, a cavity is defined between the mold 46 and the second mold 47, and a resin 49 is then injected into the cavity via a resin injection port 48 by a known injection molding method. After a cooling step, as shown in Fig. 6D, the first mold 46 and the second mold 47 are finally removed to provide an immunoassay cup 10 according to an embodiment of the present invention.

According to the above method of fabrication an immunoassay cup, the immunoassay cup 10 having integrally formed bottom 11 and side wall 14 can be obtained.

### [Second Embodiment]

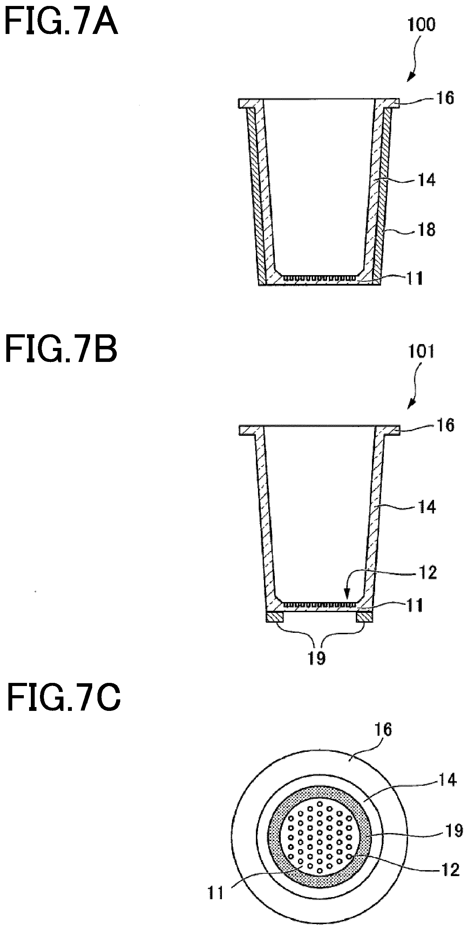

With reference to Figs. 7A to 7C, an immunoassay cup according to another embodiment of the present invention will be described. Fig. 7A is a schematic cross-sectional view of an immunoassay cup according to still another embodiment of the present invention. An immunoassay cup 100 includes a bottom 11, a side wall 14, and a light-shielding portion 18. The bottom 11 is connected to the side wall 14. The light-shielding portion 18 covers an outer surface of the side wall 14. Specifically, the light-shielding portion 18 is in contact with the outer surface of the side wall 14 and covers the entire side wall 14.

Since the bottom 11 and the side wall 14 of the immunoassay cup 100 are the same as the bottom 11 and the side wall 14 described in the first embodiment, respectively, detailed description thereof will be omitted. The immunoassay cup 100 may include a grip 16. Since the grip 16 is the same as the grip 16 described in the first embodiment, detailed description thereof will be omitted.

When immunoassay is performed by using the immunoassay cup, fluorescence is detected on the underside of the bottom of the immunoassay cup as described above. Due to the side wall of the immunoassay cup being made of a light transmitting material, external light may enter the side wall and may be detected together with fluorescence derived from the measurement target. When external light is detected together with the fluorescence derived from the measurement target, the detection sensitivity may be lowered.

The light-shielding portion 18 of the immunoassay cup 100 covers the outer surface of the side wall 14. This prevents external light from entering the immunoassay cup 100. That is, the detection sensitivity of the fluorescence emitted from the pores 12 of the immunoassay cup 100 is improved. The light-shielding portion 18 preferably covers the entire outer surface of the side wall 14. However, it may not necessarily cover the entirety, and may cover approximately 50% of the entire side wall 14, for example. When the light-shielding portion 18 does not cover the entire side wall 14, the light-shielding portion 18 is preferably disposed in a region close to the bottom 11.

The light-shielding portion 18 may be a light-shielding tape, or a light-shielding layer obtained by applying a light-shielding ink. The light-shielding tape is preferably a black light-shielding tape, and may be, for example, a PT series simple light-shielding single-sided adhesive tape manufactured by Lintec Corporation. The light-shielding tape can be adhered to and cover the entire side wall 14 to form the light-shielding portion 18. Examples of the light-shielding ink include ink containing a heat-curable resin and a carbon black. The light-shielding ink can be applied to the entire side wall 14, and then cured by applying heat or the like to form the light-shielding portion 18.

The light transmittance of the light-shielding portion 18 is preferably 50% or less, and more preferably 40% or less. The lower limit of the light transmittance of the light-shielding portion 18 is not specifically limited, and may be 0%.

The light transmittance of the light-shielding portion 18 is obtained by measuring the light transmittance in the visible light region (wavelengths: 360 to 830 nm) of the light-shielding portion 18 in the immunoassay cup by using a microspectrophotometer (for example, product name: MSV-500, manufactured by JASCO Corporation).

Figs. 7B and 7C are a schematic cross-sectional view and a bottom view, respectively, of an immunoassay cup according to still another embodiment of the present invention. The immunoassay cup 101 includes a bottom 11, a side wall 14, and a light-shielding portion 19. The bottom 11 is connected to the side wall 14. The light-shielding portion 19 is in contact with a portion of a surface of the bottom 11 on a side opposite to that in which the pores 12 are formed. The light-shielding portion 19 is disposed on a surface of the bottom 11 on a side opposite to that in which the pores 12 are formed in a region that overlaps the side wall 14 but does not overlap the pores 12. For example, as shown in Fig. 7C, the light-shielding portion 19, when viewed from the underside of the immunoassay cup 101, overlaps the side wall 14 and a curved portion of the bottom 11 which is connected to the side wall 14, but does not overlap the pores 12. The light-shielding portion 19 is disposed in a region surrounded by the outer peripheral edge of the bottom 11 and the outer peripheral edge of a region in which the pores 12 are formed.

For example, as shown in Fig. 7C, when the bottom of the immunoassay cup 101 has a circular shape and a region in which the pores 12 are formed also has a circular shape, the light-shielding portion 19 is annular.

When immunoassay is performed by using the immunoassay cup, fluorescence is detected on the underside of the bottom of the immunoassay cup as described above. Due to the side wall of the immunoassay cup being made of a light transmitting material, external light may enter the side wall, and part of the external light may be guided through the side wall to reach the bottom and may be detected together with fluorescence derived from the measurement target. When external light is detected together with the fluorescence derived from the measurement target, the detection sensitivity may be lowered.

Since the immunoassay cup 101 includes the light-shielding portion 19, it can block external light from being guided through the side wall 14 and reaching the bottom 11. Therefore, the detection sensitivity of the fluorescence emitted from the pores 12 of the immunoassay cup 101 is improved.

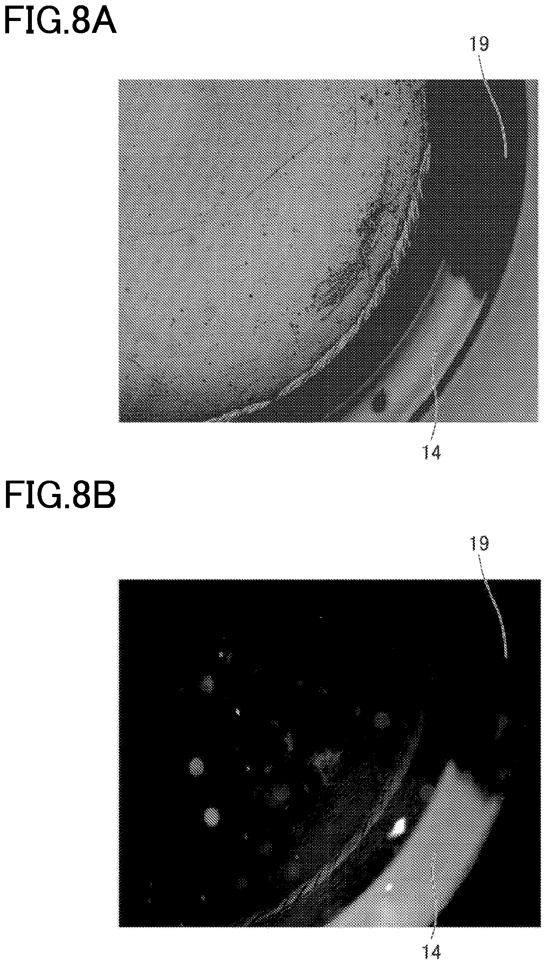

Figs. 8A and 8B are microscopic images illustrating a light-shielding portion of an immunoassay cup according to still another embodiment of the present invention. Fig. 8A is a bright field microscopic image in which the light-shielding portion 19 is disposed on part of the bottom of the immunoassay cup. Fig. 8B is a microscopic image in the fluorescence observation conditions in which the light-shielding portion 19 is disposed on part of the bottom of the immunoassay cup. Here, the pores have a minimum diameter of 4.5 µm, and the polystyrene beads (maximum diameter of 3 µm) bound to GFP as a fluorescence dye for convenience were used as the sample. The imaging conditions are as follows.

### <Imaging Conditions in Bright Field>

Apparatus: Fluorescence microscope, objective lens of 10X
Imaging site: The bottom including the pores in the immunoassay cup

### <Imaging Conditions in Fluorescence Observation>

Apparatus: Fluorescence microscope, objective lens of 10X
Excitation light source: 470 nm LED for fluorescence excitation
Measurement wavelength and exposure time: GFP (excitation wavelength 470 nm, fluorescence wavelength 527 nm) exposure time 2 seconds
Imaging site: The bottom including the pores in the immunoassay cup

The immunoassay cup shown in Fig. 8A includes a portion in which the light-shielding portion 19 is disposed, and a portion in which the light-shielding portion 19 is not disposed and the side wall 14 is exposed. As seen from the image shown in Fig. 8B, the side wall 14 appears white and bright since external light is guided through the side wall 14 in a portion in which the light-shielding portion 19 is not disposed and the side wall 14 is exposed. On the other hand, in the fluorescence measurement conditions, it is found that external light guided through the side wall 14 and reaching the bottom 11 is blocked by a portion in which the light-shielding portion 19 is provided. Further, it is also found that blocking the external light can reduce the background value and improve the signal to noise ratio. As a result, a substantially circular white area is observed in the bottom 11, which indicates that the fluorescence of one molecule of the fluorescence dye is captured. Accordingly, it is found that measurement and analysis with high accuracy can be performed by reaction of a small amount of sample.

The light-shielding portion 19 may be made of the same material as that of the light-shielding portion 18. Further, the light-shielding portion 19 may be produced by the same method as that for forming the light-shielding portion 18. Therefore, detailed description thereof will be omitted.

The light transmittance of the light-shielding portion 19 is preferably 50% or less, and more preferably 40% or less. The lower limit of the light transmittance of the light-shielding portion 19 is not specifically limited, and may be 0%. The light transmittance of the light-shielding portion 19 can be measured by the same method as that for measuring the light transmittance of the light-shielding portion 18.

Further, since the bottom 11 and the side wall 14 of the immunoassay cup 101 are the same as the bottom 11 and the side wall 14 described in the first embodiment, respectively, detailed description thereof will be omitted. The immunoassay cup 101 may include a grip 16. Since the grip 16 is the same as the grip 16 described in the first embodiment, detailed description thereof will be omitted.

The immunoassay cup according to still another embodiment of the present invention may include both the light-shielding portions 18 and 19.

### [Third Embodiment]

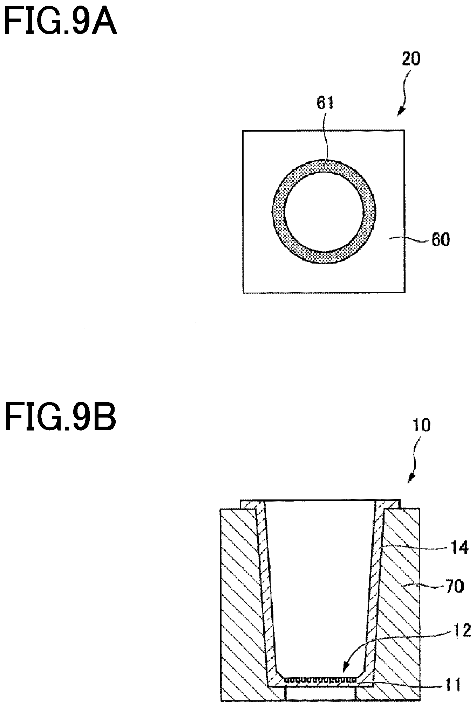

In the present embodiment, with reference to Figs. 9A and 9B, a light-shielding member used with the immunoassay cup according to still another embodiment of the present invention will be described. Fig. 9A is a top view of a sample stage according to still another embodiment of the present invention. Fig. 9B is a schematic cross-sectional view of an adapter according to still another embodiment of the present invention.

As described in the second embodiment, when the side wall of the immunoassay cup is made of a light transmitting material, external light may enter the side wall and may be detected together with fluorescence derived from the measurement target. When external light is detected together with the fluorescence derived from the measurement target, the detection sensitivity may be lowered. In order to prevent such a decrease in detection sensitivity, a sample stage 20 of the present embodiment can be used in combination with an immunoassay cup 10 according to an embodiment of the present invention.

During immunoassay, the immunoassay cup 10 according to an embodiment of the present invention is placed on the sample stage 20. Here, the immunoassay cup 10 is placed on the sample stage 20 such that a light-shielding portion 61 does not overlap a region of the immunoassay cup 10 in which the pores 12 are formed, and overlaps a region other than the region in which the pores 12 are formed. Then, fluorescence is measured by using a measurement system on a side of the sample stage 20 opposite to that on which the immunoassay cup 10 is placed.

The sample stage 20 includes a substrate 60, and the light-shielding portion 61 provided on the substrate 60. The light-shielding portion 61 can have any shape as long as it does not overlap a region of the immunoassay cup 10 in which the pores 12 are formed, and overlaps a region other than the region in which the pores 12 are formed. For example, as shown in Fig. 9A, when the bottom of the immunoassay cup 10 has a circular shape, and a region in which the pores 12 are formed also has a circular shape, the light-shielding portion 61 may have an annular shape.

The material of the substrate 60 is not specifically limited as long as it has light transmissivity, and may be, for example, a glass, quartz, or the like. The light-shielding portion 61 may be made of the same material as that of the light-shielding portion 18. Further, the light-shielding portion 61 may be produced by the same method as that for forming the light-shielding portion 18. Therefore, detailed description thereof will be omitted.

When immunoassay is performed by combining the sample stage 20 of the present embodiment with the immunoassay cup 10, the light-shielding portion 61 can block external light from being guided through the side wall 14 and reaching the bottom 11. Therefore, the detection sensitivity of the fluorescence emitted from the pores 12 of the immunoassay cup 10 is improved.

As another method of blocking external light entering the immunoassay cup 10, an adapter shown in Fig. 9B can be used. During immunoassay, while an adapter 70 according to the present embodiment holds the immunoassay cup 10, fluorescence is detected on a side of the bottom 11 opposite to that in which the pores 12 are formed by using a measurement system.

The adapter 70 is configured to cover the side wall 14 and part of the bottom 11 of the immunoassay cup 10. The adapter 70 overlaps a curved portion of the bottom 11 connected to the side wall 14, and does not overlap a region in which the pores 12 are formed.

The material for the adapter 70 may be one having light-shielding properties and able to hold the immunoassay cup 10, and is suitably aluminum subjected to alumite treatment to reduce external light reflection, but not limited thereto.

Accordingly, the adapter 70 can prevent external light from entering the immunoassay cup 10. Further, the adapter 70 can block the external light from being guided through the side wall 14 and reaching the bottom 11. Therefore, the detection sensitivity of the fluorescence derived from the measurement target substance is improved.

### [Industrial Applicability]

The immunoassay cup according to an embodiment of the present invention can be used to analyze the state of small molecular substances such as cytokines to identify rheumatoid arthritis or excessive immune reactions, and effects of nucleic acid medicines. Accordingly, a large variety of applications and development in the field of medical diagnosis are expected.

### [Reference Signs List]

- 10, 100, 101: Immunoassay cup of the present disclosure
- 50: Conventional immunoassay cup
- 11, 51: Bottom
- 12: Pore
- 13: Hydrophilic processed portion
- 14: Side wall
- 15: Upper side of the bottom
- 16: Grip
- 17: Pore bottom
- 18, 19, 61: Light-shielding portion
- 20: Sample table
- 21, 61: Dropper tip
- 22, 62: Fluorescent beads
- 23: Blood and reagent
- 24: Sealing material
- 25: Reinforcement rib
- 31: Fluorescence
- 32: Condenser lens
- 33: Measurement system
- 41: Silicon wafer
- 42: Resist pattern
- 43: Conductive layer
- 44: Nickel electrocasting
- 45: Mold
- 46: First mold
- 47: Second mold
- 48: Resin injection port
- 49: Resin
- 60: Substrate
- 70: Adapter

## Claims

1. A fluorescent immunoassay cup (10, 100, 101) for use in analysis of an immune system using an antigen-antibody reaction, in which beads (22, 62) are used as fluorescent markers, the fluorescent immunoassay cup (10, 100, 101) comprising:
a bottom (11, 51); and
a side wall (14) connected to the bottom (11, 51), wherein
the bottom (11, 51) is provided with a plurality of pores (12) each configured to accommodate a corresponding one of the beads (22, 62), and
a region between the pores (12) on an upper side of the bottom (11, 51) is formed in a planar shape, **characterized in that**
the upper side of the bottom (11, 51) and a side wall of the pores (12) are connected by a spline curve in a cross-section of the fluorescent immunoassay cup (10, 100, 101) taken perpendicular to the bottom (11, 51).

2. The fluorescent immunoassay cup (10, 100, 101) according to claim 1, wherein the bottom (11, 51) and the side wall (14) are integrally formed.

3. The fluorescent immunoassay cup (10, 100, 101) according to claim 1 or 2, wherein
an inner surface of the side wall (14) includes a curved portion in a cross-section of the fluorescent immunoassay cup (10, 100, 101) taken perpendicular to the bottom (11, 51),
an inner diameter of the side wall (14) decreases toward the bottom (11, 51), and
the inner surface of the side wall (14) is connected to the upper side of the bottom (11, 51) by a spline curve in the cross-section.

4. The fluorescent immunoassay cup (10, 100, 101) according to any one of claims 1 to 3, wherein the inner surface of the side wall (14) is hydrophobic.

5. The fluorescent immunoassay cup (10, 100, 101) according to any one of claims 1 to 4, wherein the inner surface of the pores (12) has been subjected to hydrophilic processing.

6. The fluorescent immunoassay cup (10, 100, 101) according to any one of claims 1 to 5, further comprising a reinforcement rib (25) protruding from the inner surface of the side wall (14).

7. The fluorescent immunoassay cup (100) according to any one of claims 1 to 6, further comprising a first light-shielding portion (18) that covers an outer surface of the side wall (14).

8. The fluorescent immunoassay cup (101) according to any one of claims 1 to 7, further comprising a second light-shielding portion (19), wherein
the second light-shielding portion (19) is disposed on a surface of the bottom (11, 51) on a side opposite to that in which the pores (12) are formed in a region that overlaps the side wall (14) but does not overlap the pores (12).

9. A method of producing the fluorescent immunoassay cup (10, 100, 101) according to any one of claims 1 to 8, the method comprising the steps of:
attaching a mold (45) for forming the pores (12) to a first mold (46);
combining the first mold (46) with a second mold (47) to form a cavity defined between the first mold (46) and the second mold (47); and
injecting a resin into the cavity for molding.

10. A fluorescent immunoassay method using the fluorescent immunoassay cup (10, 100, 101) according to any one of claims 1 to 8, the method comprising the steps of:
storing each of the beads (22, 62) into a corresponding one of the plurality of pores (12);
introducing a liquid, which contains a specimen as a detection target substance, and a reagent, into the plurality of pores (12);
pouring a sealing material into the fluorescent immunoassay cup (10, 100, 101) to seal an upper part of the plurality of pores (12); and
measuring fluorescence emitted from the pores (12).

## Patentansprüche

1. Fluoreszenz-Immunoassay-Becher (10, 100, 101) für eine Verwendung bei einer Analyse eines Immunsystems unter Verwendung einer Antigen-Antikörper-Reaktion, bei der Kugeln (22, 62) als fluoreszente Markierer verwendet werden, wobei der Fluoreszenz-Immunoassay-Becher (10, 100, 101) Folgendes aufweist:
einen Boden (11, 51); und
eine Seitenwand (14), die mit dem Boden (11, 51) verbunden ist, wobei
der Boden (11, 51) mit einer Vielzahl an Poren (12) versehen ist, die jeweils so aufgebaut sind, dass sie eine entsprechende der Kugeln (22, 62) unterbringen, und
ein Bereich zwischen den Poren (12) an einer oberen Seite des Bodens (11, 51) in einer ebenen Form ausgebildet ist,
**dadurch gekennzeichnet, dass**
die obere Seite des Bodens (11, 51) und eine Seitenwand der Poren (12) durch eine Splinekurve in einem senkrecht zu dem Boden (11, 51) betrachteten Querschnitt des Fluoreszenz-Immunoassay-Bechers (10, 100, 101) verbunden sind.

2. Fluoreszenz-Immunoassay-Becher (10, 100, 101) gemäß Anspruch 1, wobei der Boden (11, 51) und die Seitenwand (14) einstückig ausgebildet sind.

3. Fluoreszenz-Immunoassay-Becher (10, 100, 101) gemäß Anspruch 1 oder 2, wobei
eine Innenfläche der Seitenwand (14) einen gekrümmten Abschnitt in einem senkrecht zu dem Boden (11, 51) betrachteten Querschnitt des Fluoreszenz-Immunoassay-Bechers (10, 100, 101) umfasst,
ein Innendurchmesser der Seitenwand (14) zu dem Boden (11, 51) hin abnimmt, und
die Innenfläche der Seitenwand (14) mit der oberen Seite des Bodens (11, 51) durch eine Splinekurve in dem Querschnitt verbunden ist.

4. Fluoreszenz-Immunoassay-Becher (10, 100, 101) gemäß einem der Ansprüche 1 bis 3, wobei die Innenfläche der Seitenwand (14) mit hydrophobisch ist.

5. Fluoreszenz-Immunoassay-Becher (10, 100, 101) gemäß einem der Ansprüche 1 bis 4, wobei die Innenfläche der Poren (12) einer hydrophilischen Behandlung ausgesetzt worden ist.

6. Fluoreszenz-Immunoassay-Becher (10, 100, 101) gemäß einem der Ansprüche 1 bis 5, der des Weiteren eine Verstärkungsrippe (25) aufweist, die von der Innenfläche der Seitenwand (14) vorragt.

7. Fluoreszenz-Immunoassay-Becher (100) gemäß einem der Ansprüche 1 bis 6, der des Weiteren einen ersten Lichtabschirmungsabschnitt (18) aufweist, der eine Außenfläche der Seitenwand (14) bedeckt.

8. Fluoreszenz-Immunoassay-Becher (101) gemäß einem der Ansprüche 1 bis 7, der des Weiteren einen zweiten Lichtabschirmabschnitt (19) aufweist, wobei
der zweite Lichtabschirmabschnitt (19) an einer Oberfläche des Bodens (11, 51) an einer Seite angeordnet ist, die entgegengesetzt zu jener ist, in der die Poren (12) in einem Bereich ausgebildet sind, der mit der Seitenwand (14) überlappt aber mit den Poren (12) nicht überlappt.

9. Verfahren zum Herstellen des Fluoreszenz-Immunoassay-Bechers (10, 100, 101) gemäß einem der Ansprüche 1 bis 8, wobei das Verfahren die folgenden Schritte aufweist:
Anbringen einer Form (45) zum Ausbilden der Poren (12) an einer ersten Form (46);
Kombinieren der ersten Form (46) mit einer zweiten Form (47) zum Ausbilden eines Hohlraums, der zwischen der ersten Form (46) und der zweiten Form (47) definiert ist; und
Injizieren eines Kunststoffs in den Hohlraum zum Formgeben.

10. Fluoreszenz-Immunoassay-Verfahren unter Verwendung des Fluoreszenz-Immunoassay-Bechers (10, 100, 101) gemäß einem der Ansprüche 1 bis 8, wobei das Verfahren die folgenden Schritte aufweist:
Unterbringen von jeder der Kugeln (22, 62) in eine entsprechende der Vielzahl an Poren (12);
Einleiten einer Flüssigkeit, die eine Probe als eine Erfassungszielsubstanz enthält, und eines Reaktionsmittels in die Vielzahl an Poren (12);
Gießen eines Abdichtmaterials in den Fluoreszenz-Immunoassay-Becher (10, 100, 101) zum Abdichten eines oberen Abschnittes der Vielzahl an Poren (12); und
Messen der Fluoreszenz, die von den Poren (12) emittiert wird.

## Revendications

1. Gobelet pour test immunologique par fluorescence (10, 100, 101) destiné à être utilisé dans **l'analyse d'un système immunitaire à l'aide d'une réaction** antigène-anticorps, dans lequel des billes (22, 62) sont utilisées comme marqueurs fluorescents, le gobelet pour test immunologique par fluorescence (10, 100, 101) comprenant :
un fond (11, 51) ; et
une paroi latérale (14) reliée au fond (11, 51), dans lequel
le fond (11, 51) **est pourvu d'une pluralité de pores** (12) chacun conçu pour recevoir une bille correspondante parmi les billes (22, 62), et
une région entre les pores (12) sur un côté supérieur du fond (11, 51) est formée en une forme plate, **caractérisé en ce que**
le côté supérieur du fond (11, 51) et une paroi latérale des pores (12) sont reliés par une courbe cannelée dans une section transversale du gobelet pour test immunologique par fluorescence (10, 100, 101) prise perpendiculairement au fond (11, 51).

2. Gobelet pour test immunologique par fluorescence (10, 100, 101) selon la revendication 1, dans lequel le fond (11, 51) et la paroi latérale (14) **sont formés d'un seul tenant**.

3. Gobelet pour test immunologique par fluorescence (10, 100, 100) selon la revendication 1 ou 2, dans lequel
une surface interne de la paroi latérale (14) comprend une partie courbée dans une section transversale du gobelet pour test immunologique par fluorescence (10, 100, 101) prise perpendiculairement au fond (11, 51),
un diamètre interne de la paroi latérale (14) diminue vers le fond (11, 51), et
la surface interne de la paroi latérale (14) est reliée au côté supérieur du fond (11, 51) par une courbe cannelée dans la section transversale.

4. Gobelet pour test immunologique par fluorescence **(10, 100, 101) selon l'une quelconque** des revendications 1 à 3, dans lequel la surface interne de la paroi latérale (14) est hydrophobe.

5. Gobelet pour test immunologique par fluorescence **(10, 100, 101) selon l'une quelconque** des revendications 1 à 4, dans lequel la surface interne des pores (12) a été soumise à un traitement hydrophile.

6. Gobelet pour test immunologique par fluorescence **(10, 100, 101) selon l'une quelconque** des revendications 1 à 5, comprenant en outre une nervure de renforcement (25) dépassant de la surface interne de la paroi latérale (14).

7. Gobelet pour test immunologique par fluorescence **(101) selon l'une quelconque des** revendications 1 à 6, comprenant en outre une première partie de protection contre la lumière (18) qui couvre une surface externe de la paroi latérale (14).

8. Gobelet pour test immunologique par fluorescence **(101) selon l'une quelconque des** revendications 1 à 7, comprenant en outre une seconde partie de protection contre la lumière (19), dans lequel
la seconde partie de protection contre la lumière (19) est disposée sur une surface du fond (11, 51) sur un côté opposé à celui dans lequel les pores (12) sont formés dans une région qui chevauche la paroi latérale (14), mais ne chevauche pas les pores (12).

9. Procédé de production du gobelet pour test immunologique par fluorescence (10, 100, 101) **selon l'une quelconque des revendications 1 à 8, le procédé comprenant les étapes de** :
**fixation d'un moule** (45) pour former les pores (12) à un premier moule (46) ;
association du premier moule (46) avec un second moule (47) pour former une cavité définie entre le premier moule (46) et le second moule (47) ; et
**injection d'une résine dans la cavité pour un moulage.**

10. Procédé de test immunologique par fluorescence faisant appel au gobelet pour test immunologique par fluorescence **(10, 100, 101) selon l'une quelconque des revendications 1 à 8,** le procédé comprenant les étapes de :
stockage de chacune des billes (22, 62) dans un pore correspondant de la pluralité de pores (12) ;
**introduction d'un liquide, qui contient un échantillon en tant que substance cible de détection, et d'un réactif, dans la pluralité de pores (12)** ;
**versement d'un matériau de scellement dans le gobelet pour test immunologique** par fluorescence (10, 100, 101) pour sceller une partie supérieure de la pluralité de pores (12) ; et
mesure de la fluorescence émise depuis les pores (12).
